(19)

**Europäisches Patentamt**

**European Patent Office**

**Office européen des brevets**

(11) **EP 3 498 338 A1**

(12) **EUROPÄISCHE PATENTANMELDUNG**

(43) Veröffentlichungstag:
19.06.2019 Patentblatt 2019/25

(21) Anmeldenummer: 17208264.6

(22) Anmeldetag: 18.12.2017

(51) Int Cl.:
*A61Q 5/06* $^{(2006.01)}$        *A61K 8/87* $^{(2006.01)}$
*C08G 18/75* $^{(2006.01)}$        *C08G 18/08* $^{(2006.01)}$

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **SPEZIELLER POLYURETHANHARNSTOFF ZUR ERHÖHUNG DER WASSERBESTÄNDIGKEIT EINER GEFORMTEN FRISUR**

(57) Die Erfindung betrifft die Verwendung eines Polyurethanharnstoffs, erhältlich durch Umsetzung wenigstens

a) einer Polyisocyanat-Komponente,
b) einer polymeren Polyolkomponente,
c) einer hydrophilierenden Komponente und
d) einer aminofunktionellen Kettenverlängerer-Komponente,

wobei die Polyisocyanat-Komponente a) ≥ 75 mol-% Isophorondiisocyanat (IPDI) und die aminofunktionelle Kettenverlängerer-Komponente c) ≥ 75 mol-% Isophorondiamin (IPDA) umfasst, auf Haaren zur Verbesserung der Wasserbeständigkeit der durch Formung des Haares erhaltenen Frisur.

EP 3 498 338 A1

**Beschreibung**

Die Erfindung betrifft die Verwendung eines speziellen Polyurethanharnstoffs auf Haaren zur Verbesserung der Wasserbeständigkeit der durch Formung des Haares erhaltenen Frisur, sowie eine Methode zum wasserbeständigen und/oder waschbeständigen Formen von Haaren unter Einsatz des speziellen Polyurethanharnstoffs und eine Zusammensetzung enthaltend den speziellen Polyurethanharnstoff in besonderen Darreichungsformen.

Zur Gestaltung und Stabilisierung vielseitiger Frisuren werden Produkte eingesetzt, die als Haarfestiger bekannt sind. Haarfestiger gibt es meistens in Form von Schaumfestigern oder Haarsprays, wobei sie sich in ihrer Zusammensetzung kaum unterscheiden. Schaumfestiger werden auf das feuchte Haar als Hilfsmittel zur Modellierung der Frisur aufgetragen. Im Gegensatz hierzu, werden Haarsprays, Haarcremes, Haargele oder Haarwachse auf trockene fertig gestylte Haare zur Fixierung der Frisur für den Alltag an Luft aufgebracht.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprüh- oder Schäumvorrichtungen versprühbare Präparate vor, die aus einer alkoholischen, wässrigen oder wäßrigalkoholischen Lösung von filmbildenden natürlichen oder synthetischen Polymeren bestehen. Diese Polymere können aus der Gruppe der nichtionischen, kationischen, amphoteren oder anionischen Polymeren ausgewählt werden.

Als filmbildende Polymere werden im Stand der Technik häufig anionische oder amphotere Polymere auf Basis von Acrylaten eingesetzt. Bekannt ist aber auch die Verwendung von Polyurethanen und Polyurethanharnstoffen als Filmbildner. So sind beispielsweise in der WO 2009/118105 A1 Haarfestiger Zusammensetzungen beschrieben, die einen Polyurethanharnstoff erhalten, der durch Umsetzung eines wasserunlöslichen nicht wasserdispergierbaren isocyanatfunktionellen Polyurethanprepolymers mit einer aminofunktionellen Verbindung erhältlich ist. Die hier offenbarten Haarfestiger Zusammensetzungen sind gut zur Stabilisierung von Frisuren bis zur nächsten Haarwäsche geeignet. Allerdings können hiermit keine Frisuren nachhaltig gestaltet werden, die eine Haarwäsche überdauern würden.

Aufgabe der vorliegenden Erfindung war es daher mindestens einen Nachteil des Standes der Technik zumindest zu einem Teil zu überwinden. Weiterhin war es eine Aufgabe einen Polyurethanharnstoff bereit zu stellen, der geeignet ist bei Verwendung auf Haaren eine erhöhte Wasserbeständigkeit der hiermit geformten Frisur zu erzielen. Insbesondere war es eine Aufgabe eine Wasserbeständigkeit der mit dem Polyurethanharnstoff geformten Frisur über mehrere Waschgänge zu erzielen. Eine weitere Aufgabe war es eine Methode bereitzustellen, die es ermöglicht eine wasser- und/oder waschbeständige Frisur herzustellen.

Gelöst wurde diese Aufgabe überraschender Weise durch Verwendung eines Polyurethanharnstoffes erhältlich durch Umsetzung wenigstens der folgenden Komponenten:

   a) einer Polyisocyanat-Komponente,
   b) einer polymeren Polyolkomponente,
   c) einer hydrophilierenden Komponente und
   d) einer aminofunktionellen Kettenverlängerer-Komponente,

wobei die Polyisocyanat-Komponente a) > 75 mol-% Isophorondiisocyanat (IPDI) und die aminofunktionelle Kettenverlängerer-Komponente c) > 75 mol-% Isophorondiamin (IPDA) umfasst, auf Haaren zur Verbesserung der Wasserbeständigkeit oder der Waschbeständigkeit der durch Formung des Haares erhaltenen Frisur.

Unter Wasserbeständigkeit wird erfindungsgemäß verstanden, dass die Beständigkeit der mit dem Polyurethanharnstoff geformten Frisur gegenüber Wasser geprüft bzw. bestimmt wird. Hierbei wird das Haar in einer Spülbehandlung mindestens zu einem Teil mit flüssigem Wasser umgeben, also hierin getränkt oder gespült. Bei dem Wasser kann es sich um jede Form von flüssigem Wasser handeln, dass im Alltag mit dem Haar in Kontakt kommt. Beispiele hierfür sind Trinkwasser, Regenwasser, Schwimmbadwasser (gechlort, ozonisiert oder anderweitig keimfrei gehalten), Meerwasser oder destilliertes Wasser.

Unter Waschbeständigkeit wird erfindungsgemäß verstanden, dass die Beständigkeit der mit dem Polyurethanharnstoff geformten Frisur gegenüber tensidhaltigem Wasser geprüft bzw. bestimmt wird, wobei das Haar bei dieser Waschbehandlung komplett mit dem tensidhaltigen Wasser umgeben wird, also hierin gespült wird.

Erfindungsgemäß wird unter einer aminofunktionellen Kettenverlängerer-Komponente eine Komponente verstanden, die wenigstens eine Verbindung mit zwei isocyanatreaktiven Aminogruppen und keinen hydrophilierenden Gruppen umfasst.

Gemäß einer bevorzugten Ausführungsform der Verwendung des Polyurethanharnstoffs ist das Formen des Haares zu einer Frisur ausgewählt aus der Gruppe bestehend aus:

   a. Einem Glätten von lockigem Haar;
   b. Einem Lockeneinbringen in glattes Haar;
   c. Einem Verstärken von Locken in bereits gelocktem Haar;

d. Einer Kombination aus mindestens zwei von a. bis c..

Das Glätten von lockigem Haar unter a. kann dabei auf jede dem Fachmann bekannt Art und Weise, insbesondere mittels einer Glättvorrichtung erfolgen. Bevorzugt erfolgt das Glätten von lockigem Haar mittels eines Glätteisens, eines Glättstabes oder einer ähnlichen Glättvorrichtung, die hierfür geeignet ist. Bevorzugt ist die Glättvorrichtung so ausgestaltet, dass sie mit Hilfe von auf mindestens 150°C erhitzbaren Elementen ausgestattet ist. Bevorzugt ist die Glättvorrichtung dazu geeignet, Locken zu einem Glättungsgrad von mindestens 50%, oder bevorzugt zu mindestens 80%, oder bevorzugt zu mindestens 90% zu glätten. Die Ermittlung des Glättungsgrades erfolgt dabei durch Bestimmung der Breite einer Haarsträhne vor und nach dem Glätten mittels eines Lineals. Ein Glättungsgrad von 50%, bzw. 80%, bzw. 90% bedeutet, dass die Breite der Haarsträhne nach dem Schritt a. um 50%, bzw. 80%, bzw. 90% schmaler gegenüber der Breite vor dem Schritt a. geworden ist.

Das Lockeneinbringen in glattes Haar unter b. kann dabei auf jede dem Fachmann bekannt Art und Weise, insbesondere mittels einer Lockenerzeugungsvorrichtung erfolgen. Bevorzugt erfolgt das Lockigmachen von glattem Haar mittels eines Lockenstabes oder einer ähnlichen Lockenerzeugungsvorrichtung, die hierfür geeignet ist. Bevorzugt ist die Lockenerzeugungsvorrichtung so ausgestaltet, dass sie mit Hilfe von Locken erzeugenden Elementen, die bevorzugt leicht erwärmt werden können, beispielsweise auf Temperaturen von 40 bis 80 °C beinhaltet. Bevorzugt ist die Lockenerzeugungsvorrichtung dazu geeignet, Locken zu einem Lockungsgrad von mindestens 50%, oder bevorzugt zu mindestens 80%, oder bevorzugt zu mindestens 90% zu erzeugen. Die Ermittlung des Lockungsgrades erfolgt dabei durch Bestimmung der Länge einer Haarsträhne vor und nach dem Lockeneinbringen mittels eines Lineals. Ein Lockungsgrad von 50%, bzw. 80%, bzw. 90% bedeutet, dass die Länge der Haarsträhne nach dem Schritt b. um 50%, bzw. 80%, bzw. 90% kürzer ist als vor dem Schritt b..

Das Verstärken der Locken unter c. erfolgt bevorzugt auf die gleiche Weise, wie das Einbringen der Locken unter b. Alternativ oder zusätzlich kann das Verstärken der Locken unter c. durch Verwendung eine Polyurethanhaltigen Zusammensetzung in bevorzugt feuchtes Haar in Form der erfindungsgemäßen Verwendung erfolgen, wobei das Haar anschließend mit oder ohne Hilfsmittel getrocknet wird. Bevorzugt ist die Lockenerzeugungsvorrichtung dazu geeignet, Locken zu einem Lockungsgrad von mindestens 30%, oder bevorzugt zu mindestens 40%, oder bevorzugt zu 50% zu erzeugen. Die Ermittlung des Lockungsgrades erfolgt dabei durch Bestimmung der Länge einer Haarsträhne vor und nach dem Lockeneinbringen mittels eines Lineals. Ein Lockungsgrad von 30%, bzw. 40%, bzw. 50% bedeutet, dass die Länge der Haarsträhne nach dem Schritt b. um 30%, bzw. 40%, bzw. 50% kürzer ist als vor dem Schritt c..

In einer bevorzugten Ausführungsform der Verwendung des Polyurethanharnstoffs bleibt die Form des frisierten Haares mindestens zu 5 %, bevorzugt zu mindestens 7%, oder bevorzugt zu mindestens 10 % nach Wasserkontakt erhalten. Dabei kann der Grad der Erhaltung der Form des frisierten Haares bevorzugt stark in Abhängigkeit von der Art der Behandlung variieren. So macht es einen Unterschied, ob die Haare vor der erfindungsgemäßen Verwendung des Polyurethanharnstoffs geglättet wurden oder ob die Haare gelockt wurden. Der Grad der Erhaltung der Form des frisierten Haares wird im Folgenden auch als "styling retention" bezeichnet.

In einer bevorzugten Ausführungsform der Verwendung des Polyurethanharnstoffs bleibt die Glättung des Haares unter a. mindestens zu 50%, oder bevorzugt mindestens zu 60 %, oder bevorzugt mindestens zu mindestens 70 % oder zu mindestens 80 %, oder zu mindestens 90 % nach Wasserkontakt erhalten oder die Lockung des Haares unter b. zu mindestens 5%, oder bevorzugt zu mindestens 7 %, oder bevorzugt zu mindestens 10 % oder die Lockung unter c. zu mindestens 5%, oder bevorzugt zu mindestens 7 %, oder bevorzugt zu mindestens 10 % erhalten.

In einer bevorzugten Ausführungsform der Verwendung des Polyurethanharnstoffs, liegt der Polyurethanharnstoff in Form einer Haar-kosmetischen Zusammensetzung vor.

In einer bevorzugten Ausführungsform der Haar-kosmetischen Zusammensetzung ist diese ausgewählt aus der Gruppe bestehend aus einem Gel, einer Creme, einem Aerosole, einem Spray, einem Haarwachs oder einer Kombination aus mindestens zwei hiervon.

Gemäß einer bevorzugten Ausführungsform des Polyurethanharnstoffs zur erfindungsgemäßen Verwendung kann die Polyisocyanat-Komponente a) $\geq$ 80 mol%, bevorzugt $\geq$ 85 mol%, weiter bevorzugt 95 mol% und besonders bevorzugt 100 mol% IPDI umfassen.

Weitere - zusätzlich zu IPDI in einem molaren Anteil von weniger als 25 mol% einsetzbare - Polyisocyanate der Komponente a) sind die dem Fachmann an sich bekannten aromatischen, araliphatischen, aliphatischen oder cycloaliphatischen Polyisocyanate einer NCO-Funktionalität von $\geq$ 2.

Beispiele solcher Polyisocyanate sind 1,4-Butylendiisocyanat, 1,6-Hexamethylendiisocyanat (HDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'- und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4"-triisocyanat.

Neben den vorstehend genannten Polyisocyanaten können anteilig auch modifizierte Diisocyanate oder Triisocyanate mit Uretdion-, Isocyanurat-, Urethan-, Allophanat-, Biuret-, Iminooxadiazindion-und/oder Oxadiazintrionstruktur mit eingesetzt werden.

Bevorzugt handelt es sich um Polyisocyanate oder Polyisocyanatgemische der vorstehend genannten Art mit ausschließlich aliphatisch und / oder cycloaliphatisch gebundenen Isocyanatgruppen und einer mittleren NCO-Funktionalität der Mischung von 2 bis 4, bevorzugt von 2 bis 2,6 und besonders bevorzugt von 2 bis 2,4.

Besonders bevorzugt werden - falls neben IPDI weitere Polyisoyanate in der Komponente a) eingesetzt werden 1,6-Hexamethylendiisocyanat, die isomeren Bis(4,4'-isocyanatocyclohexyl)me-thane, sowie deren Mischungen verwendet.

Bevorzugt ist ebenfalls, wenn die polymere Polyolkomponente b) ein zahlenmittlere Molekulargewichte von $\geq$ 400 und $\leq$ 8000 g/mol, besonders bevorzugt von 600 bis 3000 g/mol eingesetzt und / oder eine mittlere OH-Funktionalitäten von 1,5 bis 6, bevorzugt von 1,8 bis 3 und besonders bevorzugt von 1,9 bis 2,1 aufweist.

Ebenfalls vorteilhaft ist, wenn die polymere Polyolkomponente b) einen Polyester, bevorzugt einen Polyester basierend auf Adipinsäure umfasst oder daraus besteht.

Mögliche Bestandteile der polymeren Polyolkomponente b) sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in b) einzeln oder in beliebigen Mischungen untereinander eingesetzt werden.

Polyesterpolyole sind beispielsweise die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele hierfür geeigneter Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols größer als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure, ganz besonders bevorzugt ist Adipinsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

In der Komponente b) können auch Hydroxylgruppen aufweisende Polycarbonate, bevorzugt Polycarbonatdiole, mit zahlenmittleren Molekulargewichten Mn von 400 bis 8000 g/mol, bevorzugt von 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art. Die Hydroxylgruppen aufweisenden Polycarbonate sind bevorzugt linear gebaut.

Ebenfalls können in der Komponente b) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrin an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Gemäß einer weiteren vorteilhaften Ausführungsform der erfindungsgemäßen Verwendung des Polyurethanharnstoffs ist vorgesehen, dass die hydrophilierende Komponente c) eine anionisch hydrophilierende Komponente und bevorzugt ein Sulfonat ist.

Geeignete anionisch bzw. potentiell anionisch hydrophilierenden Verbindungen der Komponente c) sind Verbindungen, die mindestens eine isocyanatreaktive Gruppe wie eine Hydroxylgruppe oder Amionogruppe aufweisen sowie mindestens eine Funktionalität wie z.B. -COO-M$^+$, -SO$_3$-M$^+$, - PO(O-M$^+$)$_2$ mit M$^+$ beispielsweise gleich Metallkation, H$^+$, NH$_4$$^+$, NHR$_3$$^+$, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono- und Dihydroxysulfonsäuren, sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Adduct aus 2-Butendiol und NaHSO$_3$, wie es in DE-A 2 446 440, Seite 5 - 9, Formel I-III beschrieben ist. Bevorzugte anionische oder potentiell anionische Hydrophilierungsmittel der Komponente c) sind solche der vorstehend genannten Art, die über Carboxylat- bzw. Carbonsäuregruppen und/oder Sulfonatgruppen verfügen.

Geeignete nichtionisch hydrophilierende Verbindungen der Komponente c) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38 beschrieben). Diese Verbindungen sind entweder reine Polyethylenoxidether oder gemischte Polyalkylenoxidether, wobei sie dann aber mindestens 30 mol-%, bevorzugt mindestens 40 mol-%, bezogen auf alle enthaltenen Alkylenoxideinheiten an Ethylenoxideinheiten, enthalten. Besonders bevorzugte nichtionische Verbindungen sind monofunktionelle, gemischte Polyalkylenoxidpolyether, die 40 bis 100 mol-% Ethylenoxid- und 0 bis 60 mol-% Propylenoxideinheiten aufweisen.

Zur Hydrophilierung können auch Mischungen aus anionischen bzw. potentiell anionischen Hydrophilierungsmitteln und nichtionischen Hydrophilierungsmitteln verwendet werden.

In Weiterbildung der Erfindung ist vorgesehen, dass die aminofunktionelle Kettenverlängerer-Komponente d) ≥ 85 mol%, bevorzugt ≥ 95 mol% und besonders bevorzugt 100 mol% IPDA umfassen kann.

Als weitere Bestandteile der aminofunktionelle Kettenverlängerer d) können neben IPDI weitere NH$_2$-und / oder NH-funktionelle Verbindungen eingesetzt werden.

Bevorzugt wird die Kettenverlängerung/-terminierung vor der Dispergierung in Wasser durchgeführt, dabei reagieren die Isocyanatgruppen mit dem Kettenverlängerer zu Harnstoffgruppen.

Geeignete Komponenten - die zusätzlich zu IPDA in einem molaren Anteil von weniger als 25% eingesetzt werden können - sind Di- oder Polyamine wie 1,2-Ethylendiamin, 1,2- und 1,3-Diaminopropan, 1,4-Diaminobutan, 1,6-Diaminohexan, 2,2,4- und 2,4,4-Trimethylhexamethylendiamin, 2-Methylpentamethylendiamin, Diethylentriamin, Triaminononan, 1,3- und 1,4-Xylylendiamin, $\alpha,\alpha,\alpha',\alpha'$-Tetramethyl-1,3- und -1,4-xylylendiamin und 4,4-Diaminodicyclohexylmethan und/oder Dimethylethylendiamin. Ebenfalls möglich sind Hydrazin oder sowie Hydraziden wie Adipinsäuredihydrazid.

Zur Herstellung des erfindungsgemäßen Polyurethanharnstoffs können außer den Komponten a) bis d) auch weitere Bausteine eingesetzt werden.

Beispiele sind hydroxyfunktionellen Verbindungen mit Molekulargewichten von 62 bis 399 g/mol wie beispielsweise Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit. Weiterhin können monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin.

Bevorzugt werden außer den Komponenten a) bis d) keine weiteren Bausteine zur Herstellung des Polyurethanharnstoffs zur erfindungsgemäßen Verwendung eingesetzt.

Die Herstellung des Polyurethanharnstoffs für dessen erfindungsgemäße Verwendung kann nach den dem Fachmann bekannten Verfahren in einer oder mehreren Stufe/-n in homogener oder bei mehrstufiger Umsetzung erfolgen und teilweise in disperser Phase durchgeführt werden. Bevorzugt erfolgt nach vollständig oder teilweise durchgeführter Polyaddition aus a) bis d) ein Dispergier-, Emulgier- oder Lösungsschritt. Im Anschluss erfolgt gegebenenfalls eine weitere Polyaddition oder Modifikation in disperser oder gelöster (homogener) Phase. Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren gearbeitet.

Ein weiterer Gegenstand der Erfindung ist eine Methode zum wasserbeständigen und/oder waschbeständigen Formen von Haaren zu einer Frisur mit den Schritten:

i. Behandeln des Haares mit einem Polyurethanharnstoffes, erhältlich durch Umsetzung wenigstens einer Polyisocyanat-Komponente, einer polymeren Polyolkomponente, einer hydrophilierenden Komponente und einer aminofunktionellen Kettenverlängerer-Komponente, wobei die Polyisocyanat-Komponente a) ≥ 75 mol-% Isophorondiisocyanat (IPDI) und die aminofunktionelle Kettenverlängerer-Komponente c) ≥ 75 mol-% Isophorondiamin (IPDA) umfasst;

ii. Formen des Haares zu der gewünschten Frisur;

iii. Gegebenenfalls Trocknen der Haare;

iv. Gegebenenfalls in Kontaktbringen der Haar mit Wasser.

v. Gegebenenfalls mindestens einmal Wiederholen der Schritte iii. und iv.

Unter Kontaktbringen des Haares mit Wasser unter Punkt iv. wird erfindungsgemäß verstanden, dass mindestens ein Teil des in Schritt ii. in Form gebrachten Haares in Wasser eingetaucht wird. Was unter Wasser zu verstehen ist, wurde bereits oben definiert. Hierbei ist bevorzugt ein Waschvorgang mit tensidhaltigem Wasser, auch shampoonieren genannt, in dem in Kontaktbringen der Haare mit eingeschlossen.

In einer bevorzugten Ausführungsform der Methode wird vor oder nach jedem der Schritte i. bis v. ein Waschschritt der Haare vorgenommen.

Ein weiterer Gegenstand der Erfindung ist eine wasserfeste Haar-kosmetische Zusammensetzung enthaltend einen Polyurethanharnstoff, erhältlich durch Umsetzung wenigstens

b) einer Polyisocyanat-Komponente,
c) einer polymeren Polyolkomponente,
d) einer hydrophilierenden Komponente und
e) einer aminofunktionellen Kettenverlängerer-Komponente,

wobei die Polyisocyanat-Komponente a) ≥ 75 mol-% Isophorondiisocyanat (IPDI) und die aminofunktionelle Kettenverlängerer-Komponente c) ≥ 75 mol-% Isophorondiamin (IPDA) umfasst und die Zusammensetzung in Form ausgewählt aus der Gruppe bestehend aus einem Gel, einer Creme, einem Aerosole, einem Spray, einem Haarwachs oder einer Kombination aus mindestens zwei hiervon ausgebildet ist.

Bevorzugt umfasst die Haar-kosmetische Zusammensetzung ein Lösungsmittel, ein Lösungsmittelgemisch oder ein Dispergiermittel und einen zuvor für die erfindungsgemäße Verwendung geeigneten Polyurethanharnstoff, wobei das Lösungsmittel, das Lösungsmittelgemisch oder das Dispergiermittel Wasser oder Ethanol und Wasser umfasst. Bevorzugt beinhaltet die Haar-kosmetische Zusammensetzung lediglich Wasser als Lösungsmittel bzw. Dispergiermittel.

Das Lösungsmittelgemisch kann gegebenenfalls weitere kosmetisch geeignete Lösungsmittel enthalten. Bevorzugte Lösungsmittel sind aliphatische Alkohole mit C2-4 Kohlenstoffatomen wie Isopropanol, t-Butanol, n-Butanol; Polyole wie Propylenglycol, Glycerin, Ethylenglycol und Polyolether; Aceton; unverzweigte oder verzweigte Kohlenwasserstoffe wie Pentan, Hexan, Isopentan und zyklische Kohlenwasserstoffe wie Cyclopentan und Cyclohexan; und deren Gemischen.

Für den Fall, dass die Haar-kosmetische Zusammensetzung ein Lösungsmittelgemisch aufweist, kann das Lösungsmittelgemisch ≥ 10 Gew.-% und ≤ 98 Gew.-%, bevorzugt ≥ 15 Gew.-% und ≤ 98 Gew.-%, weiter bevorzugt ≥ 20 Gew.-% und ≤ 90 Gew.-% und besonders bevorzugt ≥ 20 Gew.-% und ≤ 80 Gew.-% Ethanol umfassen.

Ebenfalls möglich ist, dass das Lösungsmittelgemisch aus Wasser und Ethanol besteht.

Bevorzugt ist für die Zusammensetzung vorgesehen, dass sie ≥ 0,1 Gew.-% und ≤ 30 Gew.-%, bevorzugt ≥ 0,1 Gew.-% und ≤ 20 Gew.-%, weiter bevorzugt ≥ 0,5 Gew.-% und ≤ 15 Gew.-% und besonders bevorzugt ≥ 0,5 Gew.-% und ≤ 10 Gew.-% des Polyurethanharnstoffs umfasst.

Bevorzugt ist auch eine Zusammensetzung, die ≥ 10 Gew.-% und ≤ 98 Gew.-%, bevorzugt ≥ 20 Gew.-% und ≤ 98 Gew.-%, weiter bevorzugt ≥ 30 Gew.-% und ≤ 98 Gew.-% und besonders bevorzugt ≥ 40 Gew.-% und ≤ 98 Gew.-% des Lösungsmittelgemisches umfasst.

Die erfindungsgemäße Zusammensetzung kann neben dem oben beschriebenen Polyurethan weitere geeignete Filmbildner enthalten, welche insbesondere auch zur Festigung und zum Styling der Haare beitragen können.

Der Anteil eines oder mehrerer weiterer Filmbildner kann von 0 bis 20 Gew-% und insbesondere 0 bis 10 Gew-% bezogen auf die gesamte Formulierung betragen.

Vorteilhaft werden der oder die weiteren Filmbildner aus der Gruppe der nichtionischen, anionischen, amphoteren und / oder kationischen Polymere und Mischungen hieraus ausgewählt.

In einer bevorzugten Ausführungsform der Haar-kosmetischen Zusammensetzung liegt die Zusammensetzung in Form ausgewählt aus der Gruppe bestehend aus einem Pumpsprays, einem Aerosols, einem Gel, einem Schaum, einem Schaumfestiger, einer Lotion, einem Wachs, einer Pomade, einem Öl, einer Milch, eine Öl in Wasser Emulsion, einer wässrigen Lösung oder einer Creme vor. Die kosmetische Zusammensetzung liegt bevorzugt in Form von Öl-in-Wasser-, Silikonin-Wasser-, Wasser-in-Öl-, Wasser-in-Silikon-, Öl-in-Wasser-in-Öl-, Wasser-in-Öl-in-Wasser-Emulsion vor.

Bevorzugt sind Wasser-in-Öl- (W/O) oder Wasser-in-Silikon-Emulsionen (W/Si), die ein oder mehrere Silikonemulgatoren (W/S) mit einem HLB-Wert $\leq$ 8 oder ein oder mehrere W/O-Emulgatoren mit einem HLB-Wert < 7 und gegebenenfalls ein oder mehrere O/W-Emulgatoren mit einem HLB-Wert > 10 enthalten, wobei HLB (nach Griffin) = 20 * (1 - $M_1$ / M), wobei $M_1$ die Molmasse des lipophilen Anteils eines Moleküls ist und M die Molmasse des gesamten Moleküls.

Die Silikonemulgatoren können vorteilhaft aus der Gruppe enthaltend Alkyldimethiconcopolyole wie z. B. Cetyl PEG/PPG 10/1 Dimethiconcopolyol (ABIL® EM 90 der Fa. Goldschmidt AG) oder Lauryl PEG/PPG- 18/18 Dimethicone (Dow Corning® 5200 der Fa. Dow Corning Ltd.) und Dimethiconecopolyole wie z. B. PEG-10 Dimethicone (KF-6017 der Fa. Shin Etsu), PEG/PPG- 18/18 Dimethicone (Dow Corning 5225C der Fa. Dow Corning Ltd.) oder PEG/PPG-19/19 Dimethicone (Dow Corning BY-11 030 der Fa. Dow Corning Ltd.) gewählt werden.

Die W/O-Emulgatoren mit einem HLB-Wert < 7 können vorteilhaft aus der Gruppe enthaltend Sorbitansstearat, Sorbitanoleat, Glycerylisostearat, Polyglyceryl-3-oleat, Pentaerythrithylisostearat, Methylglucosedioleat, PEG-7-hydriertes Ricinusoel, Polyglyceryl-4-isostearat, Hexyllaurat, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat und Wollwachsalkohol (Eucerit) gewählt werden.

Die O/W-Emulgatoren mit einem HLB-Wert > 10 können vorteilhaft aus der Gruppe enthaltend Lecithin, Trilaureth-4-Phosphat, Polysorbate-20, Polysorbate-60, PEG-22-Dodecylglycol Copolymer, Sucrosestearat und Sucroselaurat gewählt werden.

Zur Stabilisierung der erfindungsgemäßen W/O Emulsion gegen Sedimentierung oder Flockung der Wassertröpfchen kann vorteilhaft ein Ölverdicker eingesetzt werden.

Besonders vorteilhaft Ölverdicker sind organomodifizierte Tone wie organomodifizierte Bentonite (Bentone® 34 der Firma Rheox) organomodifizierte Hectorite (Bentone® 27 und Bentone® 38 der Firma Rheox) oder organomodifizierte Montmorillonit, hydrophobe pyrogene Kiesselsäure, wobei die Silanolgruppen mit Trimethylsiloxygruppen substituiert sind (AEROSIL® R812 der Firma Degussa) oder mit Dimethylsiloxygruppen oder Polydimethylsiloxan (AEROSIL® R972, AEROSIL® R974 von Degussa, CAB-O-SIL® TS-610, "CAB-O-SIL® TS-720 von Cabot), Magnesium- oder Aluminiumstearat, oder Styrol Copolymere wie zum Beispiel Styrol-Butadien-Styrol, Styrol-Isopropen-Styrol, Styrol-Ethylen/Buten-Styrol oder Styrol-Ethylen/Propen-Styrol.

Das Verdickungsmittel für die Fettphase kann in einer Menge von 0,1 bis 5 Gew.-%, bezogen auf das Gesamtgewicht der Emulsion, und besser 0,4 bis 3 Gew.-% enthalten sein.

Die wässrige Phase kann ferner Stabilisierungsmittel enthalten. Das Stabilisierungsmittel kann beispielweise Natriumchlorid, Magnesiumchlorid oder Magnesiumsulfat und deren Gemischen sein.

Öle können in W/O-, W/Si- und O/W-Emulsionen eingesetzt werden.

Wenn vorhanden, enthält die Fettphase der erfindungsgemäßen Zusammensetzung mindestens ein nicht flüchtiges Öl. Die Fettphase der Zusammensetzung kann ferner auch flüchtige Öle und Wachse enthalten. Die O/W- Zusammensetzung enthält vorteilhaft 0 bis 45 Gew.% Öle, bezogen auf das Gesamtgewicht der Zusammensetzung, und besonders vorteilhaft 0 bis 20 Gew:% Öle. Die W/O oder W/Si-Zusammensetzung enthält vorteilhaft mindestens 20 Gew.% Öle, bezogen auf das Gesamtgewicht der Zusammensetzung.

Das nicht flüchtige Öl wird vorteilhaft gewählt aus der Gruppe von mineralischer, tierischer, pflanzlicher oder synthetischer Herkunft, polaren oder unpolaren Ölen und deren Gemischen.

Polare Öle können unter der Lecithine und den Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen ausgewählt werden. Beispielweise können die Fettsäuretriglyceride gewählt werden aus der Gruppe von Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Aprikosenkernöl, Avocadoöl und dergleichen mehr.

Weitere vorteilhafte polare Öle können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Beispielsweise können die Esteröle vorzugsweise gewählt werden aus der Gruppe von Phenethylbenzoat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Diisopropyladipat, Isooctylstearat, Isononylstearat, Isononylisononanoat, 2-Ethylhexylpalmitat, 2-Ethylhexyllaurat, 2-hexyldecyllaurat, 2-Hexyldecylstearat, 2-Octyldodecylpalmitat, 2-Octyldodecylmyristat, 2-Octyldodecyllactat, 2-Diethylhexylsuccinat, Diiostearylmalat, Glyceryltriisostearat, Diglyceryltriisostearat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z.B. Jojobaöl.

Vorteilhaft können die polaren Öle gewählt werden aus der Gruppe der Dialkylether und Dialkylcarbonate, vorteilhaft sind z.B. Dicaprylylether (Cetiol® OE der Firma BASF) und/oder Dicaprylylcarbonat (beispielsweise Cetiol® CC der Firma BASF).

Es ist ferner bevorzugt, die polaren Öle aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, C12-15 Alkylbenzoat, Myristylmyristat, Isodecylneopentanoat, Triisostearin, Dipentaerythrityl Hexacaprylat/Hexacaprat, Propylenglykolmonoisostearat, Tricaprylin, Dimethylisosorbid, Butyloctylsalicylat (beispielsweise das unter der Handelsbezeichnung Hallbrite® BHB bei der Fa. CP Hall erhältliche), Hexadecylbenzoat und Butyloctylbenzoat und Gemische davon (Hallstar® AB) und/oder Diethylhexylnaphthalat (Hallbrite® TQ oder Corapan® TQ von Symrise) auszuwählen.

Das nicht flüchtige Öl kann ebenfalls vorteilhaft auch ein unpolares Öl sein, welche gewählt wird aus der Gruppe der verzweigten und unverzweigten Kohlenwasserstoffe, insbesondere Mineralöl, Vaselineöl, Paraffinöl, Squalan und Squalen, Polyolefine beispielsweise Polydecene, hydrogenierte Polyisobutene, C13-16 Isoparaffin und Isohexadecan.

Das unpolare nicht flüchtige Öl kann unter den nicht flüchtigen Silikonölen ausgewählt werden.

Von den nicht flüchtigen Silikonölen können die Polydimethylsiloxane (PDMS), die gegebenenfalls phenyliert sind, wie die Phenyltrimethicon, oder gegebenenfalls substituiert sind mit aliphatischen und/oder aromatischen Gruppen oder mit funktionellen Gruppen, beispielsweise Hydroxygruppen, Thiolgruppen und/oder Aminogruppen; mit Fettsäuren, Fettalkoholen oder Polyoxyalkylenen modifizierte Polysiloxane und deren Gemische angegeben werden.

Die erfindungsgemäße Zusammensetzung kann ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur zwischen 30°C und 200°C eine reversible Zustandsänderung fest/flüssig zeigt. Oberhalb des Schmelzpunktes wird das Wachs niedrigviskos und mischbar mit Ölen.

Der Wachs wird vorteilhaft gewählt aus den Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Schellack, Mikrowachse, Ceresin, Ozokerit, Ouricuriwachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (lkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die Wachse können in Mengen von 0 bis 10 Gew.-%, bezogen auf das Gesamtgewicht der Zusammensetzung und vorzugsweise 0 bis 5 Gew.-% enthalten sein.

Die erfindungsgemäße Zusammensetzung kann ferner ein flüchtiges Öl enthalten, das aus der Gruppe von flüchtigen Kohlenwasserstoffölen, silikonierten Ölen oder fluorierten Ölen ausgewählt wird.

Das flüchtige Öl kann in einer Menge von 0 bis 25 Gew.-%, bezogen auf das Gesamtgewicht der Emulsion, vorzugsweise 0 bis 20 Gew.-% und noch bevorzugter 0 bis 15 Gew.-% enthalten sein.

Im Sinne der vorliegenden Schrift ist ein flüchtiges Öl ein Öl, das im Kontakt mit der Haut bei Raumtemperatur und Atmosphärendruck in weniger als einer Stunde verdämpft. Das flüchtige Öl ist bei Raumtemperatur flüssig und bei Raumtemperatur und Atmosphärendruck einen Dampfdruck von 0,13 bis 40 000 Pa ($10^{-3}$ bis 300 mm Hg), vorzugsweise 1,3 bis 13 000 Pa (0,01 bis 100 mm Hg) und besonders bevorzugt 1,3 bis 13 00 Pa (0,01 bis 10 mm Hg) und einen Siedepunkt von 150 bis 260 °C und vorzugsweise 170 bis 250 °C besitzt.

Unter einem Kohlenwasserstofföl wird ein Öl verstanden, das im Wesentlichen aus Kohlenstoffatomen und Wasserstoffatomen und gegebenenfalls Sauerstoffatomen oder Stickstoffatomen gebildet wird und keine Siliziumatome oder Fluoratome enthält, wobei es auch aus Kohlenstoffatomen und Wasserstoffatomen bestehen kann; es kann aber auch Estergruppen, Ethergruppen, Aminogruppen oder Amidgruppen enthalten.

Unter einem Silikon-Öl wird ein Öl verstanden, das mindestens ein Siliziumatom und insbesondere Si-O-Gruppen enthält, wie insbesondere Polydiorganosiloxane.

Unter einem fluorierten Öl ist ein Öl zu verstehen, das mindestens ein Fluoratom enthält. Das erfindungsgemäße flüchtige Kohlenwasserstofföl kann unter den Kohlenwasserstoffölen mit einem Flammpunkt von 40 bis 102 °C, vorzugsweise 40 bis 55 °C und noch bevorzugter 40 bis 50 °C ausgewählt werden.

Beispielsweise sind die flüchtigen Kohlenwasserstoffölen solche mit 8 bis 16 Kohlenstoffatomen und deren Gemische, insbesondere verzweigte $C_{8-16}$-Alkane, wie die Isoalkane (die auch als Isoparaffine bezeichnet werden) mit 8 bis 16 Kohlenstoffatomen, Isododecan, Isodecan, Isohexadecan und beispielsweise die Öle, die unter den Handelsnamen Isopars® oder Permetyls® angeboten werden; und die verzweigten $C_{8-16}$-Ester, wie Isohexylneopentanoat und deren Gemische.

Besonders vorteilhaft sind die flüchtigen Kohlenwasserstofföle wie Isododecan, Isodecan und Isohexadecan.

Das flüchtige silikonierte Öl wird bevorzugt unter den silikonierten Ölen mit einem Flammpunkt von 40 bis 102 °C, vorzugsweise einem Flammpunkt über 55 °C und höchstens 95 °C und besonders bevorzugt im Bereich von 65 bis 95 °C ausgewählt.

Beispielsweise sind die flüchtigen silikonierten Öle geradkettigen oder cyclischen Silikonöle mit 2 bis 7 Siliziumatomen genannt werden, wobei diese Silikone gegebenenfalls Alkyl- oder Alkoxygruppen mit 1 bis 10 Kohlenstoffatomen enthalten.

Besonders vorteilhaft sind die flüchtigen silikonierten Öle wie Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemische.

Das flüchtige fluorierte Öl besitzt im Allgemeinen keinen Flammpunkt.

Beispielsweise sind die flüchtigen fluorierte Öle Nonafluorethoxybutan, Nonafluormethoxybutan, Decafluorpentan, Tetradecafluorhexan, Dodecafluorpentan und deren Gemische.

Das bevorzugte kosmetische akzeptable Medium der erfindungsgemäßen Zusammensetzung enthält Wasser und gegebenenfalls ein kosmetisch verträgliches in Wasser mischbares geeignetes organisches Lösungsmittel.

Das verwendete Wasser in der erfindungsgemäßen Zusammensetzung kann ein Blütenwasser, reines demineralisiertes Wasser, Mineralwasser, Thermalwasser und/oder Meerwasser sein.

Im Fall einer O/W Zusammensetzung als erfindungsgemäße Zusammensetzung kann der Wasseranteil im Bereich von 40 bis 95 Gew.-%, bevorzugt im Bereich von 50 bis 90 Gew.-%, ganz bevorzugt im Bereich von 60 bis 80 Gew.-% bezogen auf das Gesamtgewicht der Zusammensetzung, liegen. Im Fall einer W/O Zusammensetzung liegt der Wasseranteil im Bereich von 0 bis 60 Gew.-%, bevorzugt im Bereich von 10 bis 50 Gew.-%, ganz bevorzugt im Bereich von 30 bis 50 Gew.-% bezogen auf das Gesamtgewicht der Zusammensetzung.

Die Zusammensetzung kann ferner mit einem Treibgase aufgeschäumt werden. Die oben geschriebenen Emulsionen können durch O/W-, W/O oder W/Si-Emulgator, Verdicker (wie beispielweise Hydrodispersion) oder Feststoffe (wie beispielweise Pickeringemulsion) stabilisiert sein.

Die Zusammensetzung kann einen oder mehrere Emulgatoren bzw. oberflächenaktive Mittel enthalten.

So enthalten insbesondere Öl-in-Wasser-Emulsionen (O/W) bevorzugt mindestens einen Emulgator mit einem HLB-Wert > 7 und gegebenenfalls einen Coemulgator.

Vorteilhaft werden die folgenden nichtionischen Emulgatoren eingesetzt:

- a) Partialfettsäureester und Fettsäureester mehrwertiger Alkohole und deren ethoxylierte Derivate (z. B. Glycerylmonostearat, Sorbitanstearat, Glyceryl-Stearyl-Citrat, SucroseStearat)
- b) ethoxylierte Fettalkohole und Fettsäuren.

Besonders vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder Triethanolamin-Salze der Stearin- oder Palmitinsäure) sowie Ester der Zitronensäure wie Glycerylstearatcitrat.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemässen Zubereitungen weiter erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusoel, Polyglyceryl-4-isostearat, Acrylat/$C_{10-30}$-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

**Vorbereitung bzw. Locken oder Glättung von Haarsträhnen (Schritt 0)):**

Für die Tests wurden entweder Euro-Haarsträhnen gewellt (18 cm totale Länge, 8 cm Breite, 1,0 g +/-0,2 Gewicht) benutzt oder glatte Euro-Haarsträhnen (19 cm totale Länge, 3 cm Breite, 1,0 g +/- 0,2 Gewicht).

Vor den Tests wurden die Haarsträhnen 1 Minute mit 0,3 g eines kommerziellen Silikonfreien Shampoo (der Firma Syoss "Volume Lift Shampoo") bei 38°5 C gewaschen. Die Haarsträhnen wurden anschließend 1 Minute bei 38°C ausgespült und mit einem herkömmlichen Kamm (breite Seite) durchgekämmt. Anschließend wurden die Haarsträhnen mit Hilfe eines Föhns 1 Minute bei ca.75°C getrocknet.

**Verwendete Substanzen**

Die getesteten Substanzen werden mit folgenden Abkürzungen genannt:

Produkt A: INCI: polyurethane-48, 2 Gew.-% [erfindungsgemäss]

Produkt B: Amphomer (INCI: Ocylacrylamide/Acrylates/Butyllaminoethyl Methacrylate Copolymer), 100 % mit AMP (Amino Methylpropanol) auf pH 9,6 eingestellt, 2 Gew.-%.

Produkt C: Luviskol PVP K90, INCI: PVP (polyvinylpyrrolidon), 2 Gew.-%.

Produkt D: INCI: polyurethane-35, 2 Gew.-%

Produkt E: Kommerzielles Haar Gel, Henkel Got2B Kleber

Produkt F: Kommerzielles Haar Gel, L'Oréal Studio Line Spurenlos FX 8

**Beispiel 1: Wasserbeständigkeit von geglätteten Haaren**

**Schritt 1)**

**Vergleichsbeispiel 1:**

Nach der Vorbereitung, wie in Schritt 0) beschrieben, wurden die Haarsträhnen erneut mit dem Föhn 3 Minuten bei 75°C getrocknet. Dann wurden die Haarsträhnen 5 mal 3 Sekunde bei 230°C mit einem handelsüblichen Glätteisen geglättet und anschließend 12 Stunden bei 56 % rel.-25 Luftfeuchtigkeit bei 23 °C vollständig getrocknet.

**Beispiel 1A und 1B:** Behandlung mit Produkt A oder Produkt B

Es wurden 3 Haarsträhnen parallel gleich behandelt. Nach der Vorbereitung der Haarsträhnen, wie in Schritt 0 beschrieben, wurde jeweils 1g Produkt A oder B auf die jeweilige Haarsträhne aufgetragen. Jede der drei Haarsträhnen wurde kurz abgestreift, um das Produkt auf der Länge der jeweiligen Haarsträhne zu verteilen. Die Haarsträhnen wurden gekämmt und mit dem Föhn 3 Minuten bei 75°C getrocknet. Danach wurden sie 5 mal 3 Sekunde bei 230°C mit einem Glätteisen geglättet und anschließend 12 Stunden bei 56 % Luftfeuchtigkeit bei 23 °C vollständig getrocknet.

**Beispiel 2: Wasserbeständigkeit von gelockten Haaren**

**Vergleichsbeispiel 2:**

Nach der Vorbereitung, wie in Schritt 0) beschrieben, wurden die Haarsträhnen auf einem Kaltwellwickler gedreht und mittels eines Gummis befestigt. Die Strähnen wurden anschließend mit dem Föhn 5 Minuten bei 75°C getrocknet und anschließend 12 Stunden bei 56 % rel.-25 Luftfeuchtigkeit bei 23 °C vollständig getrocknet.

**Beispiel 2, A bis F:** Behandlung mit Produkten A bis F

Nach der Vorbereitung der Haarsträhnen, wie in Schritt 0) beschrieben, wurde jeweils 1g Produkt A B, C, D, E oder F auf die jeweilige Haarsträhne aufgetragen. Jede der drei Haarsträhnen wurde kurz abgestreift, um das Produkt auf der Länge der jeweiligen Haarsträhne zu verteilen. Die Haarsträhnen wurden gekämmt und auf einem Kaltwellwickler gedreht und mittels eines Gummis befestigt. Die Strähnen wurden dann mit dem Föhn 5 Minuten bei 75°C getrocknet und anschließend 12 Stunden bei 56 % rel.-25 Luftfeuchtigkeit bei 23 °C vollständig getrocknet.

**Prüfung der Wasserbeständigkeit**

Jede der in den Beispielen 1 und 2 behandelten Haarsträhnen wurden einzeln für 30 Sekunden vollständig in ein auf 38 °C beheiztes Wasserbad mit 2 Liter Inhalt getaucht, für 10 Sekunden abtropfen gelassen und bei Raum Temperatur an Luft vollständig für 24 h trocknen lassen.

Die Breite bzw. Länge der Strähne wurde anschließend mit einem Lineal (1 Millimeterskala) vermessen.

**Ergebnisse Beispiel 1**

| Beispiel | Eingesetzte Produkt | Breite vor/nach Glättung [cm] | Breite nach Wasserbeständigkeit Prüfung = Testbreite [cm] | Styling Retention [%] |
|---|---|---|---|---|
| 1-Vergleichsbeispiel | - | 8 / 1,4 | 5 | 45,45 |
| 1A | A | 8 / 1,4 | 1,8 | 93,9 |
| 1B | B | 8 / 1,4 | 2,8 | 78,7 |

$$Styling\ retention\ x = \frac{100 * [\text{Breite vor Glättung} - \text{Testbreite}]}{[\text{Breite vor Glättung} - \text{Breite nach Glättung}]}$$

**Ergebnisse Beispiel 2**

| Beispiel | Eingesetzte Produkt | Länge vor / nach Wickeln [cm] | Locken Länge nach der Wasserbeständigkeit Prüfung [cm] | | Unterschied* [cm] | Retention [%] |
|---|---|---|---|---|---|---|
| | | | Nass | Trocken = Testlänge | | |
| 2-Vergleichsbeispiel | Kein Polymer | 19 / 6,5 | 19 | 19 | 12,5 | 0 |
| 2A (erfindungsgemäß | A | 19 / 7,5 | 16,5 | 17,5 | 10 | 13 |
| 2B (Vergleich) | B | 19 / 5,5 | 18,5 | 18,5 | 13 | 3,7 |
| 2C (Vergleich) | C | 19 / 7,0 | 19 | 19 | 12 | 0 |
| 2D (Vergleich) | D | 19 / 6,5 | 18,5 | 18,5 | 12 | 4 |
| 2E (Vergleich) | E | 19 / 7,0 | 16,5 | 18,5 | 11,5 | 4,1 |
| 2F (Vergleich) | F | 19 / 7,5 | 18,5 | 19 | 11,5 | 0 |
| *Unterschied = Länge nach der Wasserbeständigkeit Prüfung trocken (Testlänge) - Länge direkt nach dem Wickeln | | | | | | |

$$Styling\ retention\ x = \frac{100 * [\text{Länge vor Wickeln} - \text{Testlänge}]}{[\text{Länge vor Wickeln} - \text{Länge nach Wickeln}]}$$

Anhand der Werte in den Tabellen 1 und 2 kann deutlich erkannt werden, dass die Behandlung von Haaren mit der erfindungsgemäßen Haar-kosmetischen Zusammensetzung, die den spezielle Polyurethanharnstoff beinhaltet, zu einer Verbesserung der Waschbeständigkeit und der Wasserbeständigkeit der mit dem Polyurethanharnstoff geformten Frisur führt. So sind im Vergleich zu keinem Zusatz von Polymeres aber auch zu herkömmlichen Mitteln, wie sie für die Vergleichsversuche B bis F verwendet wurden deutlich geringere "styling retention" Grade zu verzeichnen. Insbesondere bei dem Erhalt der Lockung der Haare ist dies sehr markant, da durch ohne Verwendung eines "Styling" Mittels oder durch die Verwendung von herkömmlichen Mitteln entweder keine Wasserbeständigkeit gezeigt werden konnte oder nur eine ein Drittel so hohe Wasserbeständigkeit wie mit der erfindungsgemäßen Haar-kosmetischen Zusammensetzung bzw. unter der erfindungsgemäßen Verwendung des beschriebenen Polyurethanharnstoffes.

**Patentansprüche**

1. Verwendung eines Polyurethanharnstoffs, erhältlich durch Umsetzung wenigstens

   a) einer Polyisocyanat-Komponente,
   b) einer polymeren Polyolkomponente,
   c) einer hydrophilierenden Komponente und
   d) einer aminofunktionellen Kettenverlängerer-Komponente,

   wobei die Polyisocyanat-Komponente a) ≥ 75 mol-% Isophorondiisocyanat (IPDI) und die aminofunktionelle Kettenverlängerer-Komponente c) ≥ 75 mol-% Isophorondiamin (IPDA) umfasst, auf Haaren zur Verbesserung der Wasserbeständigkeit oder der Waschbeständigkeit der durch Formung des Haares erhaltenen Frisur.

2. Die Verwendung gemäß Anspruch 1, wobei das Formen des Haares zu einer Frisur ausgewählt ist aus der Gruppe bestehend aus:

   a. Einem Glätten von lockigem Haar;
   b. Einem Lockeneinbringen in glattes Haar;
   c. Einem Verstärken von Locken in bereits gelocktem Haar;
   d. Einer Kombination aus mindestens zwei von a. bis c..

3. Die Verwendung gemäß einem der vorhergehenden Ansprüche, wobei die Form des frisierten Haares mindestens zu 5 % nach Wasserkontakt erhalten bleibt.

4. Die Verwendung gemäß Anspruch 3, wobei die Glättung des Haares unter a. mindestens zu 50% nach Wasserkontakt erhalten bleibt oder die Lockung des Haares unter b. zu mindestens 30% oder die Lockung unter c. zu mindestens 20% erhalten bleibt.

5. Die Verwendung gemäß einem der vorhergehenden Ansprüche, wobei der Polyurethanharnstoff in Form einer Haar-kosmetischen Zusammensetzung vorliegt.

6. Die Verwendung gemäß dem vorhergehenden Anspruch, wobei die Haar-kosmetische Zusammensetzung ausgewählt ist aus der Gruppe bestehend aus einem Gel, einer Creme, einem Aerosole, einem Spray, einem Haarwachs oder einer Kombination aus mindestens zwei hiervon.

7. Die Verwendung gemäß einem der vorhergehenden Ansprüche, wobei die Polyisocyanat-Komponente a) ≥ 80 mol-%, bevorzugt ≥ 85 mol-%, weiter bevorzugt 95 mol-% und besonders bevorzugt 100 mol-% Isophorondiisocyanat umfasst.

8. Die Verwendung gemäß einem der vorhergehenden Ansprüche, wobei die polymere Polyolkomponente b) ein zahlenmittleres Molekulargewicht von ≥ 400 und ≤ 8000 g/mol und /oder eine OH-Funktionalität von 1,5 bis 6 aufweist.

9. Die Verwendung gemäß einem der vorhergehenden Ansprüche, wobei die polymere Polyolkomponente b) einen Polyester, bevorzugt einen Polyester basierend auf Adipinsäure umfasst oder daraus besteht.

**10.** Die Verwendung gemäß einem der vorhergehenden Ansprüche, wobei die hydrophilierende Komponente c) eine anionisch hydrophilierende Komponente und bevorzugt ein Sulfonat ist.

**11.** Die Verwendung gemäß einem der vorhergehenden Ansprüche, wobei die aminofunktionelle Kettenverlängerer-Komponente d) $\geq$ 85 mol%, bevorzugt $\geq$ 95 mol% und besonders bevorzugt 100 mol% Isophorondiamin umfasst.

**12.** Eine Methode zum wasserbeständigen und/oder waschbeständigen Formen von Haaren zu einer Frisur mit den Schritten:

> i. Behandeln des Haares mit einem Polyurethanharnstoffes, erhältlich durch Umsetzung wenigstens einer Polyisocyanat-Komponente, einer polymeren Polyolkomponente, einer hydrophilierenden Komponente und einer aminofunktionellen Kettenverlängerer-Komponente, wobei die Polyisocyanat-Komponente a) $\geq$ 75 mol-% Isophorondiisocyanat (IPDI) und die aminofunktionelle Kettenverlängerer-Komponente c) $\geq$ 75 mol-% Isophorondiamin (IPDA) umfasst;
> ii. Formen des Haares zu der gewünschten Frisur;
> iii. Gegebenenfalls Trocknen der Haare;
> iv. Gegebenenfalls in Kontaktbringen der Haar mit Wasser.
> v. Gegebenenfalls mindestens einmal Wiederholen der Schritte iii. und iv.

**13.** Die Methode gemäß Anspruch 11, wobei vor oder nach jedem der Schritte i. bis v. ein Waschschritt der Haare vorgenommen wird.

**14.** Haar-kosmetische Zusammensetzung zur wasserfesten Gestaltung von Haaren, enthaltend einen Polyurethanharnstoff, erhältlich durch Umsetzung wenigstens

> a) einer Polyisocyanat-Komponente,
> b) einer polymeren Polyolkomponente,
> c) einer hydrophilierenden Komponente und
> d) einer aminofunktionellen Kettenverlängerer-Komponente,

wobei die Polyisocyanat-Komponente a) $\geq$ 75 mol-% Isophorondiisocyanat (IPDI) und die aminofunktionelle Kettenverlängerer-Komponente c) $\geq$ 75 mol-% Isophorondiamin (IPDA) umfasst und die Zusammensetzung in Form ausgewählt aus der Gruppe bestehend aus einem Gel, einer Creme, einem Aerosole, einem Spray, einem Haarwachs oder einer Kombination aus mindestens zwei hiervon ausgebildet ist.

Europäisches Patentamt
European Patent Office
Office européen des brevets

# EUROPÄISCHER RECHERCHENBERICHT

Nummer der Anmeldung

EP 17 20 8264

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 712 609 A1 (BAYER MATERIALSCIENCE AG [DE]) 2. April 2014 (2014-04-02)<br>* Absatz [0002] - Absatz [0006] *<br>* Absatz [0205] - Absatz [0208] *<br>* Absatz [0233] *<br>* Ansprüche *<br>----- | 1-14 | INV.<br>A61Q5/06<br>A61K8/87<br>C08G18/75<br>C08G18/08 |
| X | DE 10 2015 225967 A1 (HENKEL AG & CO KGAA [DE]) 15. September 2016 (2016-09-15)<br>* Absatz [0007] - Absatz [0008] *<br>* Absatz [0066] - Absatz [0068] *<br>* Absatz [0075] *<br>----- | 1-14 | |
| X | EP 3 020 454 A1 (COVESTRO DEUTSCHLAND AG [DE]) 18. Mai 2016 (2016-05-18)<br>* Absatz [0151] *<br>----- | 1-14 | |

RECHERCHIERTE SACHGEBIETE (IPC)

A61Q
A61K
C08G

Der vorliegende Recherchenbericht wurde für alle Patentansprüche erstellt

| Recherchenort | Abschlußdatum der Recherche | Prüfer |
|---|---|---|
| München | 26. April 2018 | S. von Eggelkraut-G. |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 17 20 8264

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-04-2018

| Im Recherchenbericht angeführtes Patentdokument | Datum der Veröffentlichung | Mitglied(er) der Patentfamilie | Datum der Veröffentlichung |
|---|---|---|---|
| EP 2712609 A1 | 02-04-2014 | EP 2712609 A1<br>TW 201424768 A<br>WO 2014048655 A2 | 02-04-2014<br>01-07-2014<br>03-04-2014 |
| DE 102015225967 A1 | 15-09-2016 | KEINE | |
| EP 3020454 A1 | 18-05-2016 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009118105 A1
- DE 2446440 A

**In der Beschreibung aufgeführte Nicht-Patentliteratur**

- Ullmanns Encyclopädie der technischen Chemie. Verlag Chemie, vol. 19, 31-38
- Microemulsions Theory and Practice. Academic Press, 1977, 21-32